# EUROPEAN PATENT APPLICATION

(11) **EP 2 127 534 A1**
(43) Date of publication of application: **02.12.2009**
(21) Application number: 07704744.7
(22) Date of filing: 24.01.2007
(51) Int. Cl.: A23G 1/02, A23G 1/32, C07D 473/10, C07D 473/12, A23K 1/14

(54) **METHOD FOR OBTAINING FIBRE-RICH COCOA WITH LOW CARBON HYDRATE AND METHYLXANTHINE CONTENTS, SUITABLE FOR ANIMAL FEED, AND RESULTING COCOA**

(71) Applicant: Natraceutical, S.A., 46004 Valencia (ES)
(72) Inventor: MOULAY, Leila, E-46920 Mislata (Valencia) (ES); BATALLER LEIVA, Esther, E-46008 Valencia (ES); RAMON VIDAL, Daniel, E-46183 L'Eliana (Valencia) (ES); GENOVÉS MARTÍNEZ, Salvador, E-46960 Aldaia (Valencia) (ES)
(74) Representative: Gonzalez-Alberto Rodriguez, Natalia
(86) International application number: PCT/ES2007/000032
(87) International publication number: WO 2008/090237

(57) **Abstract**

The invention relates to a method for obtaining fibre-rich cocoa with low carbon hydrate and methylxanthine contents and resulting cocoa powder which is suitable for animal feed. The inventive method includes the following main steps in which: the whole cocoa beans undergo cleaning, deffating, treatment with alpha-amylase, centrifugation, extraction-washing and subsequent centrifugation, separation, drying and grinding.

## Description

### FIELD OF THE ART

The invention relates to cocoa powder, more specifically it relates to a cocoa powder with high fiber content and low methylxanthine and carbohydrate content that is especially suitable for animal feed, as well as the process for obtaining said cocoa from whole cocoa beans.

### STATE OF THE ART

The use of cocoa powder as an ingredient in the formulation of food preparations for companion animals has been virtually unknown up until now due to the toxicity caused by the methylxanthines present in cocoa. However, the number of companion animals, especially cats and dogs, has currently increased and products similar to those provided to the children with whom they spend a large amount of time are to be provided to them.

In 1986 S.B. Hooser and V.R. Beasley ["Methylxanthines poisoning (chocolate and caffeine toxicosis)", in Kirk RW (ed): Current Veterinary Therapy IX. Philadelphia, WB Saunders Co, 1986, pp 191-192.] and in 1990, S.B. Beasley ["A System Affected Approach to Veterinary Toxicology". Urbana, IL; University of Illinois, 1990, pp 42-46.] identified methylxanthines as a "poison" for companion animals, but it was not until 2001 when D.B. Farbman ["Death by Chocolate? Methylxanthine Toxicosis". Veterinary Technician, March 2001, pp 146-147.] determined the amount of caffeine and theobromine present in different types of chocolate, the concentrations of caffeine and theobromine respectively being 0.174-1.460 mg/g and 4.52-25.63 mg/g present in cocoa beans, specifying that the levels can vary depending on the type of cocoa beans and of their growth conditions. It is also mentioned that the lethal dose of caffeine and theobromine for dogs is 140 mg/kg and 250-200 mg/kg respectively. For this reason, in order to consider that a cocoa powder is suitable for animal feed, it must have values of caffeine and theobromine that are lower than those considered to be toxic.

James W. Simpson has described that fiber improves the gastrointestinal health of dogs and cats ["Diet and Large Intestinal Disease in Dogs and Cats", J. Nutr. 128: 2717S-2722S, 1998.]. Based on the fact that the increase of fiber in human diet reduces hyperglycemia, PA Graham et al. ["Influence of a high fibre diet on glycaemic control and quality of life in dogs with diabetes mellitus", Journal of Small Animal Practice, 43: pp 67-73, 2002.] has studied if fiber has similar effects on the glucose metabolism of dogs with diabetes mellitus and concludes that a diet with high fiber content significantly increases glycemic level control and the quality of life of said dogs. Obesity is currently the most common disease in companion animals and it is estimated that between 25 and 44% of dogs and between 20 and 35% of cats are obese or overweight, with all the problems that this entails, therefore G. D. Sunvold and S. M. Murray mention that a diet with high fiber content aids in controlling body weight ["Nutritional Management of Weight in Dogs and Cats", Managing Gastrointestinal Health, Diabetes and Obesity- WSAVA pp 50-55, 2003.] and C. Martinez, in 2006, describes that fiber acts as a toxin chelating agent in the intestinal tract ["La Beneficiosa Fibra", Revista Alimentación Sana, Boletín 224. 2006.]. For these reasons, the second desirable requirement for a food product intended for companion animals to be considered suitable is that it has a high fiber content.

Furthermore, Patrick Nguyen et al. describe that a low carbohydrate level in the diet aids in maintaining the health of dogs by controlling their glycemic level ["Glycemic and Insulinemic Responses after Ingestion of Commercial Foods in Healthy Dogs: Influence of Food Composition", J. Nutr. 128: 2654S-2658S, 1998.]; W.J. Burkholder and J.E. Bauer describe that it aids in controlling obesity in companion animals ["Foods and techniques for managing obesity in companion animals", J. Am. Vet. Med. Assoc. 212(5): 658-62, 1998.]; and Marianne Diez et al. describe that it is the best method for achieving weight loss in obese dogs ["Weight Loss in Obese Dogs: Evaluation of a High-Protein, Low-Carbohydrate Diet" J.Nutr. 132: 1685S-1687S, 2002.]. Therefore, the third desirable characteristic for a cocoa powder intended for companion animals is that it has a low carbohydrate content.

In the field of patents, there has been an attempt to obtain cocoa powders having any of the three mentioned requirements, i.e., having a low caffeine and theobromine content, high fiber content or low carbohydrate content, but the three characteristics have not been able to be gathered in a single product.

Several methods for eliminating cocoa methylxanthines, such as caffeine and theobromine, have been proposed up until now. One of these methods involves extracting said xanthines by means of using solvents of the chlorinated hydrocarbon type, such as chloroform, ethylene dichloride or carbon tetrachloride (see for example patent documents US 1,073,441 (Robert N. Riddle) and US 1,925,326 (John Harvey Kellog et al.)). However, the use of said solvents for the extraction is not recommendable especially when foodstuffs are involved, because the residues of such solvents are completely undesirable in food products.

Another method for eliminating theobromine from cocoa beans is carrying out an extraction with water and then treating the extracted solution with an absorbent product in a separate manner, as occurs in patent document US 4,407,834 (Chiovini et al.), describing a process for eliminating xanthines from ground unroasted shelled beans, which are treated with water at 70°C-95°C for 2-3 hours. The resulting solution is separated and passed through an absorbent resin or neutralized activated carbon absorbing the xanthines. Although this method does not involve the use of organic chlorinated solvents, there are certain difficulties related to the use of absorbent materials affecting the taste of the end cocoa product.

Patent DE 102 03 568 A1 (H. von Gimborn GmbH), the object of which is to prepare a food product for domestic animals (dogs and cats) containing between 4% and 30% of cocoa powder with a maximum of 0.8% of theobromine, uses cocoa obtained from plants of the Herrania genus as well as the Theobroma grandiflorum and/or Theobroma bicolor species, because said species have a natural reduced or nil theobromine content.

Patent document DE 2 342 177 (P. Ferrero & C. S. p.A., Alba, Cuneo (Italien)) describes a process for preparing cocoa from shelled and ground cocoa beans with a particle size between 1 and 5 mm, which beans are subjected to an extraction process with water in countercurrent, from 70°C to 135°C, preferably under a pressure of 20 atmospheres, to eliminate soluble alkaloids such as caffeine and theobromine, for the purpose of using alkaloid-free cocoa in the production of beverages and other food products containing cocoa for humans, such as creams, puddings, sponge cakes or cakes.

US patent application 5,139,799 (Palson et al.) relates to a process for eliminating xanthines from cocoa by means of the ultrasonic radiation of a cocoa bean feedstock in a liquid, preferably water, up to 97% of the theobromine present in the original cocoa being able to be extracted by this means. Shelled cocoa beans are used in the 18 examples, both the representative examples of the invention and the comparative examples.

Processes are known the object of which is to obtain and purify theobromine from cocoa shells and byproducts (see for example patent document US 1,851,872 (Hermann Scheiber) and US 2,422,874 (Benjamin J. Zenlea), obtaining theobromine with a purity of up to 90-98%, the other cocoa substances being considered as waste products or products of little value.

The reduction or elimination of cocoa carbohydrates is also known. For example, patent document GB 190012956 (Jacques Apt and Paul Lohmann) describes the elimination of starch from a product obtained from ground cocoa beans the fat of which has been extracted with petroleum ether. This defatted product is boiled in water until a starch grain paste is formed, which starch grains can subsequently be eliminated and converted into caramel by heat if desired. Part or all of the fat can again be added to the starch-free defatted cocoa according to the desired dietary requirements for cocoa which is suitable for diabetics due to its lack of carbohydrates.

Document CH 622028 (Mueller Hans Dr Ing) describes an enzymatic starch degradation and the subsequent isomerization of the resulting glucose to be converted into fructose without purifying the intermediate products. By means of the process according to the invention, the starch contained in the cocoa beans is converted into a mixture of glucose and fructose, not for reducing or eliminating carbohydrates, but for preventing the addition of sweeteners that are generally used to sweeten cocoa products.

Document JP 61219339 (FUJI OIL CO LTD) describes a foodstuff formed by A) 60-97% of hardened rapeseed oil, B) 3-40% of an enzymatically treated chocolate product, and C) optionally water; and sterilizing said mixture. The chocolate component is sweet cocoa or a cocoa mass which has been treated with an enzyme such as α-amylase in an aqueous solution, the pH being adjusted to 5-10 and stirred at 50°-90°C for 15-20 minutes to obtain component (B).

In patent application PCT/ES 2006/000256 (which has not been published to date and therefore only forms part of the states of the art for purposes of novelty,) the authors of the present invention describe a process for preparing a cocoa powder with a low carbohydrate content from a solid cocoa residue obtained after a hydroalcoholic extraction of the cocoa powder.

As can be seen from the foregoing, there is a need for a cocoa-derived product having a low content of the xanthines caffeine and theobromine to make it suitable for animal feed, and further having a low carbohydrate level and a high fiber content so that it is suitable for a healthy diet.

The object of the present invention is a product obtained from whole cocoa beans complying with the desired requirements of having a low caffeine and theobromine and carbohydrate content and a high fiber content. The process for obtaining said product also forms part of the present invention.

### DETAILED DESCRIPTION OF THE INVENTION

The requirements of the final formulation of the product obtained by means of the process of the present invention consists of starting with whole cocoa beans and gradually eliminating components while at the same time the dietary fiber, mainly insoluble fiber, is conserved, the desired product thus being obtained. Several fractions of solvents with different ingredients that are susceptible to subsequent treatments for obtaining any of these ingredients are additionally obtained, which ingredients can later be used in small amounts to increase their level in the desired end product depending on its use, such as for example soluble fiber, etc.

The main use of the cocoa product obtained in the present application is cocoa for companion animals, especially dogs and cats. However, these companion animals sometimes become pets for children and the latter also use the cocoa in solidarity with their pets, in which case the product will have a low but not minimum carbohydrate level as would be the case for diabetics for example.

According to the present invention, the definition of "minimum content" is that the product in question has approximately 0-1% of that ingredient. The content of an ingredient in a "low level" is defined below, depending on said ingredient.

The selection of the starting product is crucial for reaching the objective of elaborating a product having a low caffeine and theobromine and carbohydrate content and a high fiber content, because such starting product will have a significant influence not only on the final content of the ingredients but also on the stability of said ingredients in each step of the obtaining process. To that end, unshelled cocoa beans have been chosen as starting product, because the most of the fiber which will be present in the end product is in the shell.

The differences between the process described in the aforementioned patent application PCT/ES 2006/000256 and that described in the present invention mainly relate to the raw material used, because the patent application starts from a cocoa derivative from a prior extraction with a water/alcohol solution, the result of which is that this product can have up to 80% of water and that there is a need to completely eliminate the alcohol from the solid product, because any residual amount of alcohol can partially or completely inactivate the enzyme used in the next phase of the process.

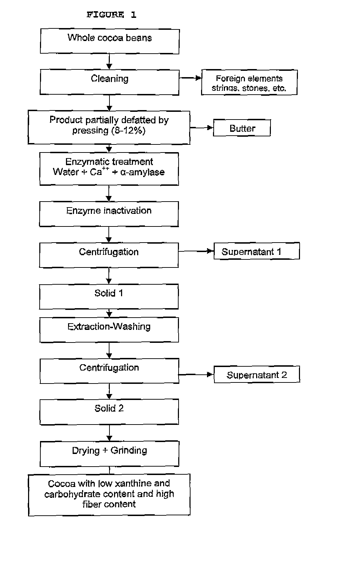

The process object of the present invention described below is schematized in Figure 1.

The cocoa cake resulting from pressing (unshelled) whole cocoa beans is used as the starting product in the present invention. This pressing is carried out to extract the cocoa butter. The starting raw material thus has a low fat content and a high fiber content. The resulting defatted product has 9-13% of fat and 4-11 % of water which is the original amount of the cocoa beans.

This solid product obtained after pressing whole cocoa beans as starting material has a theobromine concentration of 1.5-3.5%.

Table 1 shows the nutritional content of a typical cocoa powder and of the starting product for the process object of the present application.

**Table 1**

| | Typical defatted natural cocoa (%) | Cocoa product (according to the invention) (%) |
|---|---|---|
| Fat | 10-12 | 8-12 |
| Protein | 20-27 | 20-27 |
| Ashes | 5-10 | 6-10 |
| Carbohydrates | 17-22 | 20-27 |
| Moisture | 4-10 | 6-11 |
| Dietary fiber | 26-30 | 30-40 |
| Caffeine | 0.05-0.40 | 0.05-0.40 |
| Theobromine | 1.0-2.5 | 1.5-3.5 |

In the following description, the concentrations are related to the dry weight of the cocoa derivative.

The cocoa powder with low xanthine and carbohydrate content and high fiber content is obtained by the following process:
a. Cleaning of the cocoa beans
b. Defatting by pressing
c. Enzymatic treatment with an alpha-amylase
d. Enzyme inactivation
e. Centrifugation
f. Extraction-washing of the resulting solid
g. Centrifugation
h. Separation, drying, and grinding of the resulting solid

Once the defatted product is obtained, the first step is to subject the cocoa product to an enzymatic treatment to degrade the starch it contains. The cocoa product can be dried and ground until it is converted into a powder for the purpose of facilitating the access of the enzyme to small cocoa particles, but it is not necessary if the cocoa is mixed with the enzyme with sufficient water and with stirring. The fact of using a defatted cocoa product also favors the contact of the enzyme with the starch grains. In the present invention, the defatted cocoa product is mixed with water in a reactor in a cocoa produvt:water ratio of 1:6 to 1:12.

The enzymatic hydrolysis is then carried out, for which the mixture is first heated at a temperature between 50°C and 100°C, adjusting the pH of the suspension according to the instructions of the manufacturer of the enzyme so that the pH is optimal for the action of the enzyme. The pH generally varies between 5.5 and 8, acids and bases which are normally used in foodstuffs, such as citric acid, phosphoric acid, potassium hydroxide and sodium hydroxide for example, being used to adjust it.

The authors of the present invention have selected the enzyme called Termamyl 120 L (Novozymes) having an activity of 120 units per kilo (KNU/g, 1 KNU being the amount of enzyme necessary to decompose 5.26 g of starch per hour), which is used in the present invention at a concentration of 0.1 to 1.0% of enzyme with respect to the cocoa product. The α-amylase concentration is selected depending on the desired reaction time. Generally, the lower the concentration, more time is required and between 20 minutes and 4 hours of reaction are needed for the mentioned enzyme concentration. A compound providing Ca++ ions is also added, which ions act as a catalyst.

Once the reaction has been completed, the enzyme must be inactivated by increasing the temperature to 110-120°C for 15 minutes.

After the enzymatic inactivation, the mixture is centrifuged. The centrifugation takes place for 10 to 20 minutes between 4000 - 5200 rpm. The supernatant or liquid phase obtained after the centrifugation, with a high dissolved carbohydrate concentration, is separated.

The solid obtained is subjected to an extraction-washing step, which is crucial for reducing the theobromine content, with a solvent which can be water. The solvent must be added in a ratio of 1:1 to 1:12 (solid phase: water). In this phase, the solvent must be mixed with stirring and the mixture must be heated from 20°C to 100°C, for a period of 10 to 40 minutes. The heat treatment in this step is critical for the reduction of both the theobromine content and the soluble carbohydrate content. After this time, the mixture is centrifuged using the same technique used previously.

After the centrifugation, the solid product is dried and ground. The drying can be carried out from 40°C to 90°C. The product can be dried to less than 10% of moisture content, 8% for example, preferably 5%, to thus prevent microbiological growth and it is ground to a particle size of less than 200 microns, preferably less than 200 microns.

The resulting product has a xanthine content of 0.1-0.8%, specifically 0.2-0.75% of theobromine and 0.01-0.05% of caffeine, a carbohydrate content of 0.5-4.5%, a fiber content of 40-70% and a protein content of 20-30%, which makes the product nutritionally suitable.

### EXAMPLE

50 kg of whole cocoa beans were cleaned and pressed, a cocoa butter and a solid defatted cocoa product (fat content of 8-12%) being obtained, which product has been used as starting product in the example described below.

15 kg of the previously defatted cocoa product were added to 140 kg of water. The mixture was heated to 90°C for 1 hour with stirring and 8 g of CaCl2 were added as an enzymatic catalyst. The solution had a pH of 6.47. 0.2 mL of the Termamyl 120 L enzyme, of Novozyme, were used. After this time, the temperature was raised to 115°C for 20 minutes to inactivate the enzyme. A centrifugation was then carried out, after which the solid phase was separated from the liquid phase. A washing step in a 1:10 ratio of solid phase:water, which were heated at 80°C with stirring for 30 minutes, was incorporated after the centrifugation. The resulting mixture was then separated, the solid phase was dried and the product was ground to a particle size of 200 microns. The result of the chemical composition of the cocoa powder obtained in this example and representing the invention without limiting it in any aspect is shown in Table 2.

**Table 2**

| Components | | Cocoa powder Example (%) |
|---|---|---|
| Theobromine | | 0.70 |
| Caffeine | | 0.04 |
| Carbohydrates | | 3.55 |
| | Starch | 2.25 |
| | Sugars | 1.30 |
| Protein | | 22.13 |
| Fiber | | 48.00 |

## Claims

1. A cocoa powder with low xanthine and carbohydrate content and high fiber content **characterized in that** it is obtained by the following process:
a. Cleaning of the cocoa beans
b. Defatting by pressing
c. Enzymatic treatment with an alpha-amylase
d. Enzyme inactivation
e. Centrifugation
f. Extraction-washing of the resulting solid
g. Centrifugation
h. Separation, drying, and grinding of the resulting solid

2. The cocoa powder with low xanthine and carbohydrate content and high fiber content, obtained according to the process of claim 1, **characterized in that** the initial cocoa product is whole cocoa beans.

3. The cocoa powder with low xanthine and carbohydrate content and high fiber content, obtained according to the process of claim 2, **characterized in that** after subjecting the whole cocoa beans to a defatting process by pressing, the resulting cocoa product has a fat content of 8 to 13%.

4. The cocoa powder with low xanthine and carbohydrate content and high fiber content, obtained according to the process of claim 3, **characterized in that** the defatted cocoa product is mixed with water in a cocoa product:water ratio of 1:6 to 1:12 adding to the solution an alpha-amylase at an enzyme concentration of 0.1 to 1.0% with respect to the cocoa product.

5. The cocoa powder with low xanthine and carbohydrate content and high fiber content, obtained according to the process of claim 4, **characterized in that** the enzymatic starch degradation reaction is carried out at a temperature between 50°C and 100°C for a time period between 20 minutes and 4 hours.

6. The cocoa powder with low xanthine and carbohydrate content and high fiber content, obtained according to the process of claim 5, **characterized in that** the solid obtained after the enzymatic reaction has ended, after having centrifuged the mixture and having separated the supernatant, is subjected to an extraction-washing step with a solvent such as water for example in a ratio of 1:1 to 1:12 (solid:solvent), heating from 20°C to 100°C for 10 to 40 minutes.

7. The cocoa powder with low xanthine and carbohydrate content and high fiber content, obtained according to the process of claim 6, **characterized in that** the mixture is subjected to centrifugation or filtration under pressure, the supernatant and the cocoa solid which is later dried and ground being separated.

8. The cocoa powder with low xanthine and carbohydrate content and high fiber content, obtained according to the process of claim 1-7, **characterized in that** it contains a moisture of less than 10% and has a particle size of less than 200 microns.

9. The cocoa powder with low xanthine and carbohydrate content and high fiber content according to claim 8, **characterized in that** it has a xanthine content of 0.1 - 0.8%.

10. The cocoa powder with low xanthine and carbohydrate content and high fiber content according to claim 9, **characterized in that** it has a theobromine content of 0.2 - 0.75%.

11. The cocoa powder with low xanthine and carbohydrate content and high fiber content according to claim 10. **characterized in that** it has a caffeine content of 0.01 - 0.05%.

12. The cocoa powder with low xanthine and carbohydrate content and high fiber content according to claim 11, **characterized in that** it has a carbohydrate content of 0.5 - 4.5%.

13. The cocoa powder with low xanthine and carbohydrate content and high fiber content according to claim 12, **characterized in that** it has a fiber content of 40 - 70%.

14. The cocoa powder with low xanthine and carbohydrate content and high fiber content, obtained by the process according to claims 1-7 and according to claims 8-13, **characterized in that** it has a theobromine content of 0.2 - 0.75%, a caffeine content of 0.01 - 0.05%, a carbohydrate content of 0.5 - 4.5%, a fiber content of 40 - 70%, a moisture of less than 5% and it has a particle size of less than 200 microns.
